# NEW EUROPEAN PATENT SPECIFICATION

(11) **EP 3 365 316 B2**
(45) Date of publication and mention of the opposition decision: **27.04.2022**
(45) Mention of the grant of the patent: 31.07.2019
(21) Application number: 16787985.7
(22) Date of filing: 18.10.2016
(51) Int. Cl.: C07C 29/132, C07C 29/60, C07C 31/20

(54) **PROCESS FOR THE PRODUCTION OF GLYCOLS**
VERFAHREN ZUR HERSTELLUNG VON GLYCOLEN
PROCÉDÉ DE PRODUCTION DE GLYCOLS

(30) Priority: 20.10.2015 US 201562243704 P
(43) Date of publication of application: 29.08.2018
(73) Proprietor: Shell Internationale Research Maatschappij B.V., 2596 HR The Hague (NL)
(72) Inventor: MUTHUSAMY, Duraisamy, Houston, TX 77094 (US); NGUYEN, Viet, Quoc, Houston, TX 77087 (US)
(74) Representative: Shell Legal Services IP
(86) International application number: PCT/US2016/057455
(87) International publication number: WO 2017/070071

(56) References cited:
- WO-A1-2014/161852
- WO-A1-2015/028398
- WO-A1-2015/154258
- WO-A1-2016/114658

## Description

### CROSS-REFERENCE TO RELATED APPLICATIONS

This application claims priority to U.S. Provisional Application Serial No. 62/243,704 filed October 20, 2015.

### Background

Ethylene glycol and propylene glycol are valuable materials with a multitude of commercial applications, e.g. as heat transfer media, antifreeze, and precursors to polymers, such as PET. Ethylene and propylene glycols are typically made on an industrial scale by hydrolysis of the corresponding alkylene oxides, which are the oxidation products of ethylene and propylene, produced from fossil fuels.

In recent years, increased efforts have focused on producing chemicals, including glycols, from renewable feedstocks, such as sugar-based materials. The conversion of sugars to glycols can be seen as an efficient use of the starting materials with the oxygen atoms remaining intact in the desired product.

Current methods for the conversion of saccharides to sugars revolve around a hydrogenation/hydrogenolysis process as described in Angew. Chem. Int. Ed. 2008, 47, 8510-8513.

WO 2015/028398 describes a continuous process for the conversion of a saccharide-containing feedstock into glycols. In this process the saccharide-containing feedstock is contacted in a reactor with a catalyst composition comprising at least two active catalytic components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum and compounds and complexes thereof.

An important aim in this area is the provision of a process that is high yielding in desirable products, such as mono-ethylene glycol (MEG) and mono-propylene glycol (MPG), and that can be sustained with such yields over time.

However, the present inventors have found that, when using catalysts known in the art, such as those taught in WO 2015/028398, for the hydrogenation/ hydrogenolysis of saccharide-containing feedstock, significant catalyst deactivation was found to occur over time.

It would, therefore, be advantageous to provide a process for the production of glycols from saccharide-containing feedstocks in which catalyst deactivation is reduced or avoided.

### Summary

Accordingly, in one aspect, a process for the production of glycols is provided, the process comprising:
contacting a saccharide-containing feedstock with hydrogen in the presence of a solvent and a catalyst composition, wherein the solvent comprises water and from 25% to 75% by weight, based on the total weight of the solvent, of one or more alcohols selected from methanol, ethanol, 1-propanol, iso-propanol, glycerol, erythritol, threitol, sorbitol, and a combination thereof, and wherein the catalyst composition comprises at least two active catalytic components, the active catalyst components comprising, as a first active catalyst component, one or more materials selected from transition metals from groups 8, 9 or 10 or compounds thereof, with catalytic hydrogenation capabilities; and, as a second active catalyst compo-nent, one or more materials selected from tungsten, molybdenum, lanthanum, tin or compounds or complexes thereof.

### Brief Description of the Drawings

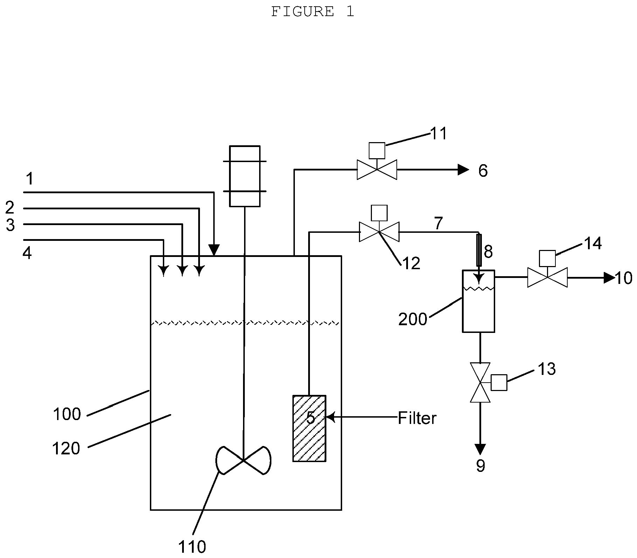

Figure 1 is a schematic representation of the experimental apparatus used in Examples 1 and 2.

### Detailed Description

The present disclosure relates to hydrogenation/ hydrogenolysis processes for the production of ethylene and propylene glycols in which a saccharide-containing feedstock is contacted with hydrogen in the presence of a solvent and a catalyst composition, wherein the solvent comprises water and from 25% to 75% by weight, based on the total weight of the solvent, of one or more alcohols selected from methanol, ethanol, 1-propanol, iso-propanol, glycerol, erythritol, threitol, sorbitol, and a combination thereof. These processes allow the hydrogenation/ hydrogenolysis reactions to be carried out while minimising any deactivation of the catalyst composition used therefor.

The present inventors have surprisingly found that the amounts of ethylene and propylene glycols yielded by known hydrogenation/hydrogenolysisprocessesare reduced over time, due to deactivation of the catalytic compositions.
It is postulated, without wishing to be bound by any theory, that such deactivation is, at least in part, caused by the deposition of water-insoluble carbonaceous materials on one or more of the active catalytic components of the catalyst composition. Such carbonaceous materials are generally believed to formed as a result of the decomposition of at least a portion of the saccharide-containing feedstock and/or the formation of reaction by-products. By utilising a solvent comprising water and from 25% to 75% by weight, based on the total weight of the solvent, of one or more alcohols selected from methanol, ethanol, 1-propanol, iso-propanol, glycerol, erythritol, threitol, sorbitol, and a combination thereof as a reaction medium, such carbonaceous materials are more readily dissolved therein and thus, more readily hydrogenated, thereby minimising or preventing the fouling of one or more of the active catalytic components of the catalyst composition.

Suitable saccharide-containing feedstocks comprise at least onesaccharide selected from the group consisting of monosaccharides, disaccharides,oligosaccharides, polysaccharides and a combination thereof. Exam pies of suitable polysaccharides include cellulose, hemicelluloses, starch, glycogen, chitin and any combination thereof. Examples of monosaccharides include glucose, fructose, etc. If the feedstock comprises oligosaccharides or polysaccharides, it is preferable that it is subjected to pre-treatment before being fed to a reactor in a form that can be converted in the processes of the present disclosure. Suitable pre-treatment methods are known in the art and include, but are not limited to, one or more of sizing, drying, blending, grinding, washing, de-watering, solids removal, steeping, milling, hot water treatment, steam treatment, hydrolysis (e.g. acid-catalysed hydrolysis, enzymatic hydrolysis), pyrolysis, thermal treat-ment, chemical treatment, biological treatment, purification, etc.

Suitable saccharide-containing feedstocks may be derived from grains such as corn, wheat, millet, oats, rye, sorghum, barley or buckwheat, from rice, from pulses such as soybean, pea, chickpea or lentil, from bananas and/or from root vegetables such as potato, yam, sweet potato, cassava and sugar beet, or any combinations thereof. A preferred source of a saccharide-containing feedstock is corn.

Preferably, a saccharide-containing feedstock supplied to a reactor after any optional pre-treatment comprises one or more saccharides selected from glucose, sucrose, and starch. Suitably, a saccharide-containing feedstock is generally supplied to a reactor as a solution, a suspension or slurry in the solvent, or in one or more components of the solvent.

Solvents suitable for use herein comprise water and from 25% to 75% by weight, based on the total weight of the solvent, of one or more alcohols selected from methanol, ethanol, 1-propanol, iso-propanol, glycerol, erythritol, threitol, sorbitol, and a combination thereof.

The solvent, or one or more of the components of the solvent (e.g. water and the one or more alcohols), may be added to the reactor in one or more separate feed streams. Similarly, the solvent, or one or more components thereof, may be added to the saccharide-containing feedstock before it enters the reactor.

The saccharide-containing feedstock is contacted with hydrogen in the presence of a catalyst composition comprising at least two active catalytic components, said active catalyst components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin and compounds and complexes thereof.

The saccharide-containing feedstock may be contacted with hydrogen in the presence of a catalyst composition in one or more reactors in parallel or in series.

The catalyst composition and the components contained therein may be heterogeneous or homogeneous with respect to the solvent or solvents present in the reactor. The catalyst composition may also contain both heterogeneous and homogeneous components.

Depending on the physical state of the catalyst composition and any components contained therein, they may be preloaded into the reactor or, if they are in liquid form or present as a solution or slurry in a solvent, they may be fed into the reactor as required in a continuous or discontinuous manner.

The catalyst composition comprises at least two active catalytic components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin or compounds or complexes thereof.

Preferably, the first active catalyst component consists of one or more of the group selected from iron, cobalt, nickel, ruthenium, rhodium, palladium, iridium and platinum. This component may be present in the elemental form or as a compound. It is also suitable that this component is present in chemical combination with one or more other ingredients in the catalyst system. It is required that the first active catalyst component has catalytic hydrogenation capabilities and it is capable of catalysing the hydrogenation of material present in the reactor.

Preferably, the second active catalyst component comprises of one or more compound, complex or elemental material comprising tungsten, molybdenum, lanthanum or tin. More preferably, the second active catalyst component comprises one or more material selected from the list consisting of tungstic acid, molybdic acid, ammonium tungstate, ammonium metatungstate, ammonium paratungstate, tungstate compounds comprising at leastone Group I or II element, metatungstate compounds comprising at least one Group I or II element, paratungstate compounds comprising at least one Group I or II element, heteropoly compounds of tungsten, heteropoly compounds of molybdenum, tungsten oxides, molybdenum oxides and combinations thereof. The metal component is suitably in a form other than a carbide, nitride, or phosphide. Preferably, the second active catalyst component comprises one or more compound, complex or elemental material selected from those containing tungsten or molybdenum.

Preferably, at least one of the active catalyst components is supported on a solid support. In this embodiment, any other active catalyst component may be present in either heterogeneous or homogeneous form. Said any other active catalyst component may also be supported on a solid support. In one embodiment of the invention, the first active catalyst component is supported on one solid support and the second active catalyst component is supported on a second solid support which may comprise the same or different material. In another embodiment, both active catalyst components are supported on one solid support.

The solid supports may be in the form of a powder or in the form of regular or irregular shapes such as spheres, extrudates, pills, pellets, tablets, monolithic structures. Alternatively, the solid supports may be present as surface coatings, for example on the surfaces of tubes or heat exchangers. Suitable solid support materials are those known to the skilled person and include, but are not limited to aluminas, silicas, zirconium oxide, magnesium oxide, zinc oxide, titanium oxide, carbon, activated carbon, zeolites, clays, silica alumina and mixtures thereof.

Suitably, the weight ratio of the first active catalyst component to the second active catalyst component is in the range of from 0.02:1 to 3000:1, preferably in the range of from 0.1:1 to 100:1, on the basis of the weight of metal present in each component.

The weight ratio of the first active catalyst component (based on the amount of metal in said component) to sugar is suitably in the range of from 1:100 to 1:1000. The weight ratio of the second active catalyst component (based on the amount of metal in said component) to sugar is suitably in the range of from 1:10 to 1:100.

If more than one reactor is used in series, a catalyst composition may optionally be present in the second and any subsequent reactors. If a catalyst composition is present in the second and any subsequent reactor, the catalyst composition used in each of the reactors may be the same or different. Additionally, the weight ratio of the active catalyst components may be varied between the first and second reactors (and any subsequent reactors) and it may be advantageous to alter the composition of the catalyst systems between the reactors to suit the different feed streams provided to each reactor. Suitably, reaction conditions, particularly temperature and pressure, can be varied between the reactors if more than one reactor is used. This can lead to a more tailored process to suit the different constituents of the feeds provided to each reactor.

The reaction temperature at which the saccharide-containing feedstock is contacted with hydrogen in the presence of the catalyst composition described herein is suitably at least 130°C, preferably at least 150°C, more preferably at least 170°C, most preferably at least 190°C. The temperature in the reactor is suitably at most 300 °C, preferably at most 280°C, more preferably at most 270°C, even more preferably at most 250°C. Preferably, the reactor is heated to a temperature within these limits before addition of the saccharide-containing feedstock and is maintained at such a temperature as the reaction proceeds.

The pressure in the reactor or reactors in which the saccharide-containing feedstock is contacted with hydrogen in the presence of the catalyst composition described herein is suitably at least 1 MPa, preferably at least 2 MPa, more preferably at least 3 MPa. The pressure in the reactor is suitably at most 15 MPa, preferably at most 12 MPa, more preferably at most 10 MPa, most preferably at most 8 MPa. Preferably, the reactor is pressurised to a pressure within these limits by addition of hydrogen before addition of any saccharide-containing feedstock and is maintained at such a pressure as the reaction proceeds through ongoing addition of hydrogen.

The processes of the present disclosure takes place in the presence of hydrogen. Preferably, the processes take place in the absence of air or oxygen. In order to achieve this, it is preferable that the atmosphere in the reactor be evacuated and replaced an inert gas, such as nitrogen, and then with hydrogen repeatedly, after loading of any initial reactor contents, before the reaction starts.

Suitable reactors include stirred tank reactors, slurry reactors, ebulated bed reactors, jet flow reactors, mechanically agitated reactors, bubble columns, such as slurry bubble columns and external recycle loop reactors. The use of these reactors allows dilution of the saccharide-containing feedstock and intermediates to an extent that provides high degrees of selectivity to the desired glycol product (mainly ethylene and propylene glycols), such as by effective back-mixing.

The residence time in the reactor is suitably at least 1 minute, preferably at least 2 minutes, more preferably at least 5 minutes. Suitably the residence time in the reactor is no more than 5 hours, preferably no more than 2 hours, more preferably no more than 1 hour.

Having generally described the invention, a further understanding may be obtained by reference to the following examples, which are provided for purposes of illustration and are not intended to be limiting unless otherwise specified.

### Examples

### Experimental Apparatus:

The apparatus used to perform the experiments shown in examples 1 and 2 is schematically represented in Figure 1. A one-liter Hastelloy-C autoclave (100) was equipped with automatic controls for the control of reactor temperature, back-pressure, liquid level, and stirrer speed. Feed line (1) was equipped with a gas flow meter and was used to provide a continuous flow of hydrogen gas into the reactor. Each of the liquid feed lines (2) and (3) was equipped with a pump and a mass flow meter. Liquid feed line (4) was equipped with a pump capable of pumping slurry feeds, such as a suspension of starch in water. These feed lines were used to continuously feed the carbohydrate feed in the form of a solution or slurry, the sodium meta-tungstate (NaMT) retro-Aldol catalyst, a solvent, and optionally a pH control agent. The excess gas pressure present in the reactor (100) was vented via line (6) by the use of back-pressure control valve (11). Filter element (5) was used to retain the heterogeneous hydrogenation catalyst inside reactor (100) while allowing the flow of the liquid product, which was controlled by valve (12), via line (7). Line (8) was a coaxial in-line product cooler with the ability to cool down the product mixture to or below room temperature. The gas-saturated product effluent passing through line (8) was set up to flow into gas-liquid separator (200). Valve (14) was used to control the pressure in the gas-liquid separator (200). Valve (13) was used to control the level in gas-liquid separator (200). Samples of the product stream were taken via line (9) for analysis. Experimental results are reported in the following examples.

### Materials

Raney-nickel (WR Grace Raney-nickel 2800), sodium meta-tungstate (NaMT), sodium bicarbonate (NaHCO₃), ethylene glycol (EG), 1,2-propylene glycol (PG), 1,2-butanediol (12BDO), and glycerol were purchased from Sigma-Aldrich chemical company. A sample of Kingsford-Knorr corn starch was purchased from a grocery store in Amsterdam.

### Analytical Methods

1. pH measurements were made using Thermo Scientific's Orion Star A211 bench top pH meter and the meter was calibrated with standard buffer solutions in the 4-10 pH range.
2. HPLC analysis of the liquid samples was performed using the following method and conditions:
   Liquid Chromatography System - Shimadzu
      Controller - SCL-10Avp
      Pump - LC-20AD
      Degasser - DGU-20A 5r
      Autosampler - SIL-10AF
      Column Oven - CTO-20AC
      UV detector - SPD-20AV
      RI detector - RID-10A
   HPLC instrument conditions:
      Column: Bio-Rad Aminex HPX-87H (300 mm x 7.8 mm)
      Flow Rate: 0.6 ml/minute
      Column Oven: 30C
      Injection Volume: 10 µl
      UV Detector: @320 NM
      RI Detector: mode - A; range - 100
      Run Time: 66 minute
      Mobile Phase: 5 mM Sulfuric Acid in water
Standard solutions containing glucose, sorbitol, ethylene glycol (EG), 1,2-propylene glycol (PG), 1,2-butanediol (12BDO), glycerol, erythritol, threitol, xylitol, etc. were prepared using water as the solvent at various concentrations. These solutions were analyzed to create the HPLC calibration curves. Samples were analyzed, with or without further dilution, and the calibration factors were applied to calculate the concentrations of the various products present in the experimental samples.

### Example 1 (inventive): Production of glycols from starch using Raney-Ni and sodium meta-tungstate (NaMT) catalysts in water-methanol solvent mixture

In this example, 27.95 grams of a sample of WR Grace Raney-nickel 2800 was added to the autoclave (Reactor 100) as slurry in 500 ml of water. The autoclave level control was set up to maintain 500 ml of liquid holdup volume in the reactor. A continuous flow of hydrogen was provided and the reactor pressure was controlled in the range of 6894.7 kPa (1000 psig). The catalyst was washed with deionized water at a rate of 5 ml per minute until the pH of the reactor effluent reached near neutrality. The catalyst was then activated by ramping up the temperature to 100°C and holding at temperature overnight.

Preparation of water-washed starch sample: A batch washing procedure was employed in which 750 grams of Kingsford-Knorr corn starch was mixed with 7.5 kg of deionized water in a 2-gallon plastic container. The resulting slurry was shaken for a minimum of two hours and then filtered under vacuum using a Buchner funnel and filter paper. The filter cake was allowed to air dry overnight, transferred onto a plastic tray placed inside a fume hood, and then crushed to a powder. The
powder was allowed to further dry under the normal laboratory humidity conditions and then transferred to a sealed container for storage. The dry weight of the starch was determined by drying a small sample in a vacuum oven at a temperature of 50-60°C until no further weight loss occurred in successive weight measurements.

A slurry of the water-washed Kingsford-Knorr corn starch was prepared in a solvent mixture containing methanol and water in 50:50 weight ratio with a starch concentration of 10.28 wt% on dry weight basis. The slurry was added to a feed vessel that was equipped with a mixer in order to maintain the suspension with a uniform concentration. The slurry was pumped into Reactor 100 with the use of a specially designed positive displacement pump that is suitable for slurry feeding. A solution of 2.00 wt% sodium meta-tungstate, the retro-Aldol catalyst, in water was prepared and loaded into another feed vessel. This solution was pumped into reactor 100 with the use of a HPLC pump.

The following experimental conditions for the reactor were used: combined slurry plus liquid feed rate in the range of 320-355 grams per hour, reaction temperature of 230°C, pressure of 10342.1 kPa (1500 psig), hydrogen flow rate of 25 standard liters per hour, and stirrer RPM of 1500. The experimental conditions are additionally given in Table 1. Samples of the product stream taken via line (9) were analyzed by pH probe and HPLC to determine pH and the concentrations of the various products. The experimental results are given in Table 2.

At the end of the run the solids present in Reactor 100 were recovered as slurry in water, and the slurry was separated into a Raney-nickel fraction and a
fraction consisting of the oxides of tungsten. The Raney-Ni fraction was filtered, washed with water, and dried in air to obtain 33.71 grams of a passivated sample of the spent Raney-Ni catalyst.

**Table 1**

| | **Autoclave Conditions** | | | **Feed Rate [G/min]** | | **Conc. in Rxn Mixture, %wt** | | |
|---|---|---|---|---|---|---|---|---|
| **Run Time (h)** | **Temp (°C)** | **Pressure (kPa) [PSIG]** | **RPM** | **Starch Conc** | **NaMT** | **Starch** | **NaMT** | **pH** |
| 5.1 | 230 | 10342.1 [1500] | 1500 | 4.43 | 0.91 | 8.53 | 0.3408 | 3.2 |
| 7.1 | 230 | 10342.1 [1500] | 1500 | 4.43 | 0.91 | 8.53 | 0.3408 | 3.2 |
| 22.6 | 230 | 10342.1 [1500] | 1500 | 4.43 | 0.91 | 8.53 | 0.3408 | 3.4 |
| 24.6 | 230 | 10342.1 [1500] | 1500 | 4.43 | 0.91 | 8.53 | 0.3408 | 3.4 |
| 31.0 | 230 | 10342.1 [1500] | 1500 | 4.43 | 1.45 | 7.75 | 0.4932 | 3.2 |
| 46.9 | 230 | 10342.1 [1500] | 1500 | 4.43 | 1.45 | 7.75 | 0.4932 | 3.3 |
| 49.4 | 230 | 10342.1 [1500] | 1500 | 4.43 | 1.45 | 7.75 | 0.4932 | 3.4 |
| 55.0 | 230 | 10342.1 [1500] | 1500 | 4.45 | 1.39 | 7.84 | 0.4760 | 3.5 |
| 67.4 | 230 | 10342.1 [1500] | 1500 | 4.42 | 1.39 | 7.82 | 0.4785 | 3.6 |
| 84.7 | 230 | 10342.1 [1500] | 1500 | 4.42 | 1.45 | 7.74 | 0.4940 | 4.0 |
| 89.2 | 230 | 10342.1 [1500] | 1500 | 4.42 | 1.45 | 7.74 | 0.4940 | 4.1 |
| 113.2 | 230 | 10342.1 [1500] | 1500 | 4.42 | 1.45 | 7.74 | 0.4940 | 4.0 |
| 116.2 | 230 | 10342.1 [1500] | 1500 | 4.47 | 1.45 | 7.77 | 0.4899 | 3.9 |
| 131.7 | 230 | 10342.1 [1500] | 1500 | 4.47 | 1.45 | 7.77 | 0.4899 | 3.9 |

Yields are defined as weight of product recovered divided by weight of glucose times 100%. Based on weight of starch, the yields are defined as weight of product recovered divided by weight of starch times 90%. The average of MEG yields is 34.9 %w/w and the average of the total glycols (MEG, MPG, and 1,2-BDO combined) is 39.6%w/w. For the majority of the run time these yields are nearly constant without significant decline in yield during time.

**Table 2 - product yields, Example 1**

| | ***Wt% Yields - Calculated on the Basis of Glucose Content of Starch** | | | | | | | | | |
|---|---|---|---|---|---|---|---|---|---|---|
| **Run time (h)** | **Glucose** | **Sorbitol** | **C4 Sugars** | **Glycerol** | **Isosorb** | **EG** | **PG** | **HA** | **1,2BDO** | **Total** |
| 5.1 | 0.0 | 13.8 | 11.9 | 5.1 | 1.9 | 35.9 | 5.3 | 0.4 | 1.1 | 75.5 |
| 7.1 | 0.0 | 7.5 | 9.3 | 3.6 | 1.6 | 47.1 | 4.1 | 0.3 | 1.2 | 74.8 |
| 22.6 | 0.0 | 9.1 | 9.4 | 2.7 | 1.7 | 42.1 | 2.9 | 0.2 | 1.0 | 69.3 |
| 24.6 | 0.0 | 9.2 | 9.4 | 2.6 | 1.6 | 40.9 | 2.7 | 0.2 | 1.0 | 67.6 |
| 31.0 | 0.0 | 10.0 | 10.5 | 2.7 | 1.7 | 41.9 | 2.8 | 0.1 | 0.9 | 70.7 |
| 46.9 | 0.0 | 10.2 | 9.9 | 2.4 | 1.9 | 38.7 | 2.6 | 0.0 | 0.9 | 66.6 |
| 49.4 | 0.0 | 10.9 | 10.7 | 2.3 | 2.3 | 43.8 | 2.6 | 0.2 | 1.5 | 74.4 |
| 55.0 | 0.0 | 9.9 | 10.7 | 2.0 | 1.6 | 50.8 | 2.8 | 0.2 | 2.0 | 80.0 |
| 67.4 | 0.0 | 6.9 | 7.7 | 1.7 | 1.7 | 42.4 | 2.3 | 0.1 | 1.9 | 64.7 |
| 84.7 | 0.0 | 5.4 | 6.5 | 1.8 | 1.9 | 44.6 | 3.5 | 0.6 | 3.2 | 67.5 |
| 89.2 | 0.0 | 4.2 | 5.2 | 1.5 | 1.6 | 40.4 | 3.3 | 0.6 | 3.2 | 60.0 |
| 113.2 | 0.0 | 2.9 | 3.4 | 1.3 | 1.9 | 11.3 | 3.4 | 6.7 | 1.7 | 32.6 |
| 116.2 | 0.0 | 0.0 | 3.4 | 1.5 | 2.0 | 7.0 | 3.1 | 7.6 | 2.2 | 26.8 |
| 131.7 | 0.0 | 0.0 | 2.9 | 0.0 | 1.8 | 2.1 | 1.6 | 6.3 | 1.0 | 15.8 |
| *C4 sugars include erythritol and threitol; isosorb = isosorbide, HA = hydroxyacetone; 1,2-BDO = 1,2-butanediol | | | | | | | | | | |

### Example 2 (Comparative): Production of glycols from starch using Raney-Ni and sodium meta-tungstate (NaMT) catalysts in water solvent

In this example, 29.63 grams of a sample of WR Grace Raney-nickel 2800 was added to the autoclave (Reactor 100) as slurry in 500 ml of water. The autoclave level control was set up to maintain 500 ml of liquid holdup volume in the reactor. A continuous flow of hydrogen was provided and the reactor pressure was controlled in the range of 6894.7 kPa (1000 psig). The catalyst was washed with deionized water at a rate of 5 ml per minute until the pH of the reactor effluent reached near neutrality. The catalyst was then activated by ramping up the temperature to 100°C and holding at temperature overnight.

A slurry of the water-washed Kingsford-Knorr corn starch was prepared in deionized water with a starch concentration of 10.94 wt% on dry weight basis. The slurry was added to a feed vessel that was equipped with a mixer in order to maintain the suspension with a uniform concentration. The slurry was pumped into Reactor 100 with the use of a specially designed positive displacement pump that is suitable for slurry feeding. A solution of 2.00 wt% sodium meta-tungstate, the retro-Aldol catalyst, in water was prepared and loaded into another feed vessel. This solution was pumped into Reactor 100 with the use of a HPLC pump.

The following experimental conditions for Reactor 100 were used: combined slurry plus liquid feed rate of 397 grams per hour for the first 52 hours and 154 grams per hour for the last 27 hours, reaction temperature of 230°C, pressure of 10342.1 kPa (1500 psig), hydrogen flow rate of 25 standard liters per hour, and stirrer RPM of 1250 or 1700. The experimental conditions are additionally given in Table 3. Samples of the product stream taken via line (9) were analyzed by pH probe and HPLC to determine pH and the concentrations of the various products. The experimental results are given in Table 4.

At the end of the run the solids present in Reactor 100 were recovered as slurry in water, and the slurry was separated into a Raney-nickel fraction and a fraction consisting of the oxides of tungsten. The Raney-Ni fraction was filtered, washed with water, and dried in air to obtain 33.0 grams of a passivated sample of the spent Raney-Ni catalyst.

**Table 3**

| | **Autoclave Conditions** | | | **Feed Rate [G/min]** | | **Conc in Rxn Mixture, %wt** | | |
|---|---|---|---|---|---|---|---|---|
| **Run Time(h)** | **Temp (°C)** | **Pressure (kPa) [PSIG]** | **RPM** | **Starch Conc** | **NaMt** | **Starch** | **NaMT** | **pH** |
| 4.5 | 230 | 10342.1 [1500] | 1250 | 5.67 | 0.95 | 9.37 | 0.2870 | 3.1 |
| 5.5 | 230 | 10342.1 [1500] | 1250 | 5.67 | 0.95 | 9.37 | 0.2870 | 3.1 |
| 20.8 | 230 | 10342.1 [1500] | 1250 | 5.67 | 0.95 | 9.37 | 0.2870 | 3.1 |
| 23.0 | 230 | 10342.1 [1500] | 1250 | 5.67 | 0.95 | 9.37 | 0.2870 | 3.1 |
| 26.7 | 230 | 10342.1 [1500] | 1250 | 5.67 | 0.95 | 9.37 | 0.2870 | 3.1 |
| 29.0 | 230 | 10342.1 [1500] | 1250 | 5.67 | 0.95 | 9.37 | 0.2870 | 3.2 |
| 44.7 | 230 | 10342.1 [1500] | 1250 | 5.67 | 0.95 | 9.37 | 0.2870 | 3.2 |
| 46.9 | 230 | 10342.1 [1500] | 1250 | 5.67 | 0.95 | 9.37 | 0.2870 | 3.3 |
| 49.5 | 230 | 10342.1 [1500] | 1250 | 5.67 | 0.95 | 9.37 | 0.2870 | 3.6 |
| 52.5 | 230 | 10342.1 [1500] | 1250 | 5.67 | 0.95 | 9.37 | 0.2870 | 3.3 |
| 54.8 | 230 | 10342.1 [1500] | 1700 | 2.07 | 0.49 | 9.32 | 0.3828 | 3.3 |
| 56.2 | 230 | 10342.1 [1500] | 1700 | 2.07 | 0.49 | 9.32 | 0.3828 | 3.3 |
| 71.1 | 230 | 10342.1 [1500] | 1700 | 2.07 | 0.49 | 9.32 | 0.3828 | 3.2 |
| 75.4 | 230 | 10342.1 [1500] | 1700 | 2.07 | 0.49 | 9.32 | 0.3828 | 3.1 |
| 79.4 | 230 | 10342.1 [1500] | 1700 | 2.07 | 0.49 | 9.32 | 0.3828 | 3.1 |

The average of MEG yields is 29.3% and the average of total glycols (MEG, MPG, and 1,2-BDo combined) is 34.6%. Severe catalyst deactivation is apparent, as glycol yields are low and yields of the corresponding hydroxyl-ketone intermediates are higher.

**Table 4 - product yields, Example 2 (Comparative)**

| | ***Wt% Yields - Calculated on the Basis of Glucose Content of Starch** | | | | | | | | | |
|---|---|---|---|---|---|---|---|---|---|---|
| **Run time (h)** | **Glucose** | **Sorbitol** | **C4 Sugars** | **Glycerol** | **Isosorb** | **EG** | **PG** | **HA** | **1,2BDO** | **Total** |
| 4.5 | 0.4 | 25.2 | 13.4 | 4.2 | 0.0 | 27.8 | 4.0 | 0.3 | 1.2 | 76.5 |
| 5.5 | 0.3 | 23.1 | 13.5 | 3.8 | 0.0 | 31.6 | 4.1 | 0.3 | 1.3 | 77.9 |
| 20.8 | 0.3 | 16.6 | 13.3 | 1.7 | 0.0 | 45.8 | 2.5 | 0.3 | 1.5 | 82.2 |
| 23.0 | 0.4 | 14.6 | 13.3 | 1.6 | 0.0 | 48.0 | 2.3 | 0.5 | 1.7 | 82.4 |
| 26.7 | 0.4 | 12.7 | 12.9 | 1.7 | 0.0 | 50.5 | 2.2 | 0.4 | 1.9 | 82.8 |
| 29.0 | 0.4 | 11.2 | 12.6 | 1.6 | 0.0 | 52.2 | 2.3 | 0.5 | 2.3 | 83.0 |
| 44.7 | 0.4 | 3.7 | 7.2 | 1.4 | 0.0 | 45.6 | 3.7 | 1.7 | 4.3 | 67.9 |
| 46.9 | 0.3 | 2.1 | 4.8 | 1.0 | 0.0 | 34.1 | 3.1 | 1.2 | 3.7 | 50.3 |
| 49.5 | 0.3 | 1.3 | 3.2 | 0.7 | 0.0 | 23.2 | 2.2 | 1.1 | 2.7 | 34.7 |
| 52.5 | 0.4 | 1.2 | 3.5 | 0.8 | 0.0 | 21.2 | 2.7 | 3.2 | 2.8 | 35.7 |
| 54.8 | 0.4 | 1.5 | 3.9 | 1.1 | 0.0 | 26.2 | 4.9 | 2.6 | 5.0 | 45.6 |
| 56.2 | 0.3 | 1.3 | 3.6 | 1.2 | 0.0 | 26.3 | 5.0 | 3.0 | 5.4 | 46.0 |
| 71.1 | 0.2 | 0.3 | 1.2 | 0.0 | 0.0 | 4.6 | 1.4 | 4.2 | 1.7 | 13.5 |
| 75.4 | 0.2 | 0.0 | 0.9 | 0.0 | 0.0 | 1.6 | 0.8 | 4.1 | 0.9 | 8.5 |
| 79.4 | 0.2 | 0.0 | 0.8 | 0.0 | 0.0 | 1.1 | 0.6 | 4.1 | 0.8 | 7.6 |
| *C4 sugars include erythritol and threitol; isosorb = isosorbide, HA = hydroxyacetone; 1,2-BDO = 1,2-butanediol | | | | | | | | | | |

### Example 3: Analysis of spent catalyst

A sample of the spent Raney-Ni catalyst recovered from Example-1 was extracted with methanol solvent in a Soxhlet extractor and the methanol solution was evaporated to obtain a carbonaceous residue. The amount of the residue accounted for 0.4% wt of the recovered catalyst, indicating a relatively clean catalyst with respect to fouling by the decomposition products of starch.

### Example 4 (Comparative): Analysis of spent catalyst

A sample of the spent Raney-Ni catalyst recovered from Example-2 was extracted with methanol solvent in a Soxhlet extractor and the methanol solution was evaporated to obtain a carbonaceous residue. The amount of the residue accounted for 16.2% wt of the recovered catalyst, indicating a catalyst that was heavily fouled by the decomposition products of starch.

The Examples clearly demonstrate that the use of an alcoholic solvent in the reaction medium has the benefits of 1) higher average glycols product yields, 2) longer run time before the catalyst deactivates due to fouling by the heavy ends produced in the reaction, and 3) lack of fouling by carbonaceous materials thus making it easier to regenerate the catalyst.

## Claims

1. A process for the production of glycols comprising:
contacting a saccharide-containing feedstock with hydrogen in the presence of a catalyst composition and a solvent,
wherein the solvent comprises water and from 25% to 75% by weight, based on the total weight of the solvent, of one or more alcohols selected from methanol, ethanol, 1-propanol, iso-propanol, glycerol, erythritol, threitol, sorbitol, and a combination thereof, and
wherein the catalyst composition comprises at least two active catalytic components, the active catalyst components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin or compounds or complexes thereof.

2. A process as claimed in Claim 1, wherein the saccharide-containing feedstock comprises one or more saccharides selected from glucose, sucrose, and starch.

3. A process as claimed in Claim 1 or claim 2, wherein the glycols comprise monoethylene and monopropylene glycols.

## Patentansprüche

1. Vorgang für die Herstellung von Glykolen, der Folgendes umfasst:
Inberührungbringen eines saccharidhaltigen Ausgangsmaterials mit Wasserstoff in der Gegenwart einer Katalysatorzusammensetzung und eines Lösungsmittels,
wobei das Lösungsmittel Wasser und von 25 bis 75 Gew.-%, basierend auf dem Gesamtgewicht des Lösungsmittels, eines oder mehrerer Alkohole umfasst, ausgewählt aus Methanol, Ethanol, 1-Propanol, Isopropanol, Glycerin, Erythrit, Threit, Sorbit und einer Kombination davon, und
wobei die Katalysatorzusammensetzung wenigstens zwei aktive katalytische Komponenten umfasst, wobei die aktiven Katalysatorkomponenten als eine erste aktive Katalysatorkomponente ein oder mehrere Materialien, ausgewählt aus Übergangsmetallen der Gruppen 8, 9 oder 10 oder Verbindungen davon mit katalytischen Hydrierungsfähigkeiten, und als eine zweite aktive Katalysatorkomponente ein oder mehrere Materialien umfassen, ausgewählt aus Wolfram, Molybdän, Lanthan, Zinn oder Verbindungen oder Komplexen davon.

2. Vorgang nach Anspruch 1, wobei das saccharidhaltige Ausgangsmaterial ein oder mehrere Saccharide umfasst, ausgewählt aus Glucose, Saccharose und Stärke.

3. Vorgang nach Anspruch 1 oder 2, wobei die Glykole Monoethylen- und Monopropylenglykole umfassen.

## Revendications

1. Procédé de production de glycols comprenant :
la mise en contact d'une charge d'alimentation contenant des saccharides avec de l'hydrogène en présence d'une composition de catalyseur et d'un solvant,
le solvant comprenant de l'eau et de 25 % à 75 % en poids, par rapport au poids total du solvant, d'un ou plusieurs alcools choisis parmi le méthanol, l'éthanol, le 1-propanol, l'isopropanol, le glycérol, l'érythritol, le thréitol, le sorbitol, et une combinaison de ceux-ci, et
la composition de catalyseur comprenant au moins deux composants catalytiques actifs, lesdits composants de catalyseur actifs comprenant, comme un premier composant de catalyseur actif, une ou plusieurs matières choisies parmi les métaux de transition des groupes 8, 9 ou 10 ou des composés de ceux-ci, ayant des capacités d'hydrogénation catalytique ; et, comme un second composant de catalyseur actif, une ou plusieurs matières choisies parmi le tungstène, le molybdène, le lanthane, l'étain ou des composés ou complexes de ceux-ci.

2. Procédé selon la revendication 1, dans lequel la charge d'alimentation contenant des saccharides comprend un ou plusieurs saccharides choisis parmi le glucose, le saccharose et l'amidon.

3. Procédé selon la revendication 1 ou la revendication 2, dans lequel les glycols comprennent des monoéthylèneglycols et des monopropylèneglycols.
